(19) Europäisches Patentamt
European Patent Office
Office européen des brevets

(11) Veröffentlichungsnummer: **0 230 235 B1**

# (12) EUROPÄISCHE PATENTSCHRIFT

(45) Veröffentlichungstag der Patentschrift: **11.07.90**

(51) Int. Cl.[5]: **C07D 335/02**, C07D 309/04, A01N 43/16, A01N 43/18

(21) Anmeldenummer: **87100212.7**

(22) Anmeldetag: **09.01.87**

(54) **Cyclohexenonderivate, Verfahren zur ihrer Herstellung und ihre Verwendung als herbizide Mittel.**

(30) Priorität: **11.01.86 DE 3600642**

(43) Veröffentlichungstag der Anmeldung: **29.07.87 Patentblatt 87/31**

(45) Bekanntmachung des Hinweises auf die Patenterteilung: **11.07.90 Patentblatt 90/28**

(84) Benannte Vertragsstaaten: **AT BE CH DE ES FR GB GR IT LI NL SE**

(56) Entgegenhaltungen:
**EP-A- 0 131 875**
**EP-A- 0 136 647**
**EP-A- 0 136 702**
**DE-A- 3 314 816**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Jahn, Dieter, Dr., Burgunder Weg 8, D-6803 Edingen-Neckarhausen(DE)**
Erfinder: **Keil, Michael, Dr., Fontanestrasse 4, D-6713 Freinsheim(DE)**
Erfinder: **Kolassa, Dieter, Dr., Moltkestrasse 8, D-6700 Ludwigshafen(DE)**
Erfinder: **Schirmer, Ulrich, Dr., Berghalde 79, D-6900 Heidelberg(DE)**
Erfinder: **Würzer, Bruno, Dr., Rüdigerstrasse 13, D-6701 Otterstadt(DE)**
Erfinder: **Meyer, Norbert, Dr., Dossenheimer Weg 22, D-6802 Ladenburg(DE)**
Erfinder: **Jung, Johann, Prof. Dr., Hardenburgstrasse 19, D-6703 Limburgerhof(DE)**
Erfinder: **Rademacher, Wilhelm, Dr., Austrasse 1, D-6703 Limburgerhof(DE)**

EP 0 230 235 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Cyclohexenonderivate mit herbizider Wirkung, Mittel auf der Grundlage solcher Cyclohexenonderivate sowie Verfahren zu ihrer Herstellung und Anwendung als Herbizide.

Die herbizide Wirkung von Oximethern mit Cyclohexenonstruktur und nichtaromatischen Substituenten in Position 5 ist aus EP 71 707 bekannt.

Es ist weiterhin bekannt, daß bestimmte 2-Acyl-3-hydroxycyclohex-2-en-1-one das Pflanzenwachstum regulieren (EP 123 001 und EP 126 713).

Bekannt sind auch Cyclohexenonderivate der unten angegebenen Formel I, worin jedoch $R^3$ Wasserstoff bedeutet (EP-A 131 875).

Es wurde gefunden, daß Cyclohexenonderivate der Formel

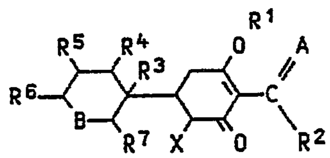

(I)

in der die Substituenten folgende Bedeutung haben:
A Sauerstoff oder ein Rest $NOR^8$, wobei $R^8$ eine $C_1$–$C_4$-Alkylgruppe, eine $C_3$–$C_4$-Alkenylgruppe, eine $C_3$–$C_4$-Alkinylgruppe, eine ein- bis dreifach durch Halogenatome substituierte $C_2$–$C_4$-Alkyl- oder $C_2$–$C_4$-Alkenylgruppe, eine $C_2$–$C_4$-Alkoxyalkylgruppe oder ein Chlorthienylrest,
B ein Sauerstoff- oder Schwefelatom oder eine Sulfon- oder Sulfoxidgruppe,
X Wasserstoff oder eine Methoxycarbonylgruppe,
$R^1$ Wasserstoff, eine $C_2$–$C_{18}$-Alkylcarbonylgruppe, ein Benzoylrest oder ein anorganisches oder orgakisches Kation,
$R^2$ eine $C_1$–$C_4$-Alkylgruppe,
$R^3$ eine $C_1$–$C_3$-Alkoxygruppe oder eine $C_1$–$C_3$-Alkylthiogruppe, wenn $R^4$ eine Hydroxygruppe bedeutet,
$R^4$ eine $C_1$–$C_3$-Alkoxygruppe oder eine $C_1$–$C_3$-Alkylthiogruppe, wenn $R^3$ eine Hydroxygruppe bedeutet, oder
$R^3$, $R^4$ unabhängig voneinander Hydroxy, Chlor, Brom, $C_2$–$C_4$-Alkylcarbonyloxy, $C_2$–$C_4$-Alkylcarbonylthio oder gemeinsam ein Sauerstoffatom,
$R^5$, $R^6$ Wasserstoff, eine Methylgruppe oder gemeinsam eine Methylenoxyethylengruppe und
$R^7$ Wasserstoff oder eine Methylgruppe, eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Ferner sind zahlreiche Verbindungen auch in Gramineenkulturen wie Weizen und Reis selektiv und bekämpfen gleichzeitig unerwünschte Gräser. Sie sind vergleichbaren Verbindungen biologisch überlegen.

Beispiele für $R^1$ in Formel I sind: Wasserstoff, Acetyl, Propionyl, Butyryl, Pivaloyl, Palmitoyl, Stearoyl, Benzoyl, Natrium, Kalium, Ammonium, Calcium, Magnesium, Trialkylammonium, Tetraalkylammonium, Trialkylbenzylammonium, Trialkylsulfonium, Trialkylsulfoxonium.

Beispiele für $R^2$ in Formel I sind: Methyl, Ethyl, n-Propyl, i-Propyl, n-Butyl, i-Butyl.

Beispiele für $R^3$ und R4 sind: Alkoxy wie Methoxy, Ethoxy, n-Propoxy, i-Propoxy, Alkylthio wie Methylthio, Ethylthio, n-Propylthio, Hydroxy, Brom, Chlor, Alkylcarbonylthio wie Methylcarbonylthio, Ethylcarbonylthio, Acetoxy, Propionyloxy, Butyryloxy, daneben können R3 und R4 auch gemeinsam eine Epoxigruppe bilden.

Beispiele für $R^8$ sind: Methyl, Ethyl, n-Propyl, i-Propyl, Allyl, (E)-2-Butenyl, 2-Fluoreth-1-yl, 2-Chloreth-1-yl, (E)-3-Chlor-2-propenyl, 1,1,2-Trichlorprop-1-en-3-yl, Chlorthienyl, Propargyl, Methoxymethyl, Methoxyethyl.

Die Cyclohexenonderivate der Formel I mit den Bedeutungen A = $NOR^8$ und $R^1$ = Wasserstoff können durch Umsetzung der Formel

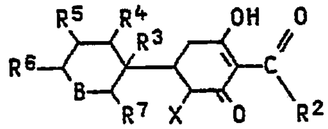

in der $R^2$, $R^3$, $R^4$, $R^5$, $R^6$, $R^7$ und X die obengenannten Bedeutungen haben, mit Alkoxyammoniumverbindungen der Formel $R^8$ $ONH_3Y$, in der Y ein Anion bedeutet, erhalten werden. Hierzu setzt man die beiden Reaktionsteilnehmer, z.B. in einem Lösungsmittel in Gegenwart einer Base bei einer Temperatur, zwischen 0°C und dem Siedepunkt des Lösungsmittels miteinander um. Geeignete Basen sind beispielsweise Carbonate, Hydrogencarbonate, Acetate, Alkoholate, Oxide oder Hydroxide von Alkali- oder Erdalkalimetallen, insbesondere Natrium, Kalium, Magnesium, Calcium oder organische Basen, wie Pyridin oder tertiäre Amine.

Als Lösungsmittel sind beispielsweise Dimethylsulfoxid, Alkohole wie Methanol, Ethanol, Isopropanol, Benzol, Toluol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann durch Einengen der Mischung, Verteilung des Rückstandes in Methylenchlorid/Wasser und Abdestillieren des Lösungsmittels unter vermindertem Druck isoliert werden.

Die Verbindungen der Formel I mit den Bedeutungen A = $NOR^8$ und $R^1$ = Wasserstoff können außerdem durch Umsetzen der Verbindungen der Formel I mit den Bedeutungen A = Sauerstoff und $R^1$ = Wasserstoff mit entsprechenden Alkoxyaminen der Formel $R^8$-$ONH_2$, ein einem geeigneten Verdünnungsmittel erhalten werden; eine geeignete Reaktionstemperatur liegt im allgemeinen zwischen 15 und 70°C. Das Alkoxylamin kann in Form einer wäßrigen Lösung eingesetzt werden; je nach dem verwendeten Lösungsmittel für den anderen Reaktionsteilnehmer erhält man ein ein- oder zweiphasiges Reaktionsgemisch.

Geeignete Lösungsmittel für die Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Alkalimetallsalze der Verbindungen I können durch Behandeln der Verbindungen mit alkalischen Verbindungen wie Natrium- oder Kaliumhydroxid oder- alkoholat in wäßriger Lösung oder in einem organischen Lösungsmittel, wie Methanol, Ethanol, Aceton, Toluol erhalten werden.

Andere Metallsalze, z.B. die Mangan-, Kupfer-, Zink-, Eisen-, Calcium-, Magnesium- und Bariumsalze können aus den Natriumsalzen in üblicher Weise (durch Umsetzung mit Salzen der Metalle mit anorganischen Säuren) hergestellt werden, ebenso können die Ammonium- und Phosphoniumsalze mittels Ammoniak, Phosphonium-, Sulfonium- oder Sulfoxoniumhydroxiden hergestellt werden.

Die Verbindungen der Formel I mit der Bedeutung A = Sauerstoff können z.B. aus den entsprechenden Cyclohexan-1,3-dionen II

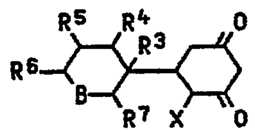 (II).

nach literaturbekannten Methoden (Tetrahedron Lett.29, 2491 (1975)) hergestellt werden.

Es ist auch möglich, Verbindungen der Formel I mit der Bedeutung A = Sauerstoff über die Zwischenstufe der Enolester, die bei der Umsetzung von Verbindungen der Formel II mit Säurechloriden in Gegenwart von Basen anfallen und anschließend mit bestimmten Imidazol- oder Pyridinderivaten umgelagert werden, herzustellen (JP-OS 79/063052).

Zu den Verbindungen der Formel II gelangt man nach literaturbekannten Methoden, wie sie z.B. in DE-OS 32 19 490 aufgeführt sind.

Es ist jedoch auch möglich die Substituenten $R^3$ und $R^4$ auf einer späteren Synthesestufe, bevorzugt auf der Stufe der Verbindungen der Formel I mit der Bedeutung A = Sauerstoff einzuführen.

Geeignete Vorprodukte sind Verbindungen der Formel

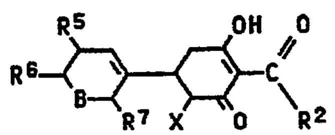

bei denen literaturbekannte Reaktionen an der Doppelbindung ausgeführt werden können, z.B. Addition von Halogenen, Halogenwasserstoffen, Mercaptanen, Thiolcarbonsäuren, Mercaptocarbonsäuren, Epoxidierung.

Die so erhaltenen Epoxy-Derivate können als Ausgangsprodukte für weitere Synthesen durch Öffnung des Oxiran-Ringes dienen, wie z.B. Umsetzung mit Mercaptanen, Wasser oder Alkoholen gegebenenfalls unter Katalyse von Säuren oder Basen.

Die Verbindungen der Formel I mit B = SO oder $SO_2$ können auch durch Oxidation von entsprechenden Vorstufen, in denen der Schwefel eine niedrigere Oxidationsstufe besitzt, erhalten werden. Oxidationsmittel sind beispielsweise Sauerstoff, Ozon, Peroxyverbindungen, wie Wasserstoffperoxide, Persäuren, Hydroperoxide, Halogene, anorganische Halogenverbindungen, wie Hypochlorit, Chlorat, Stickstoffverbindungen, wie Salpetersäure und Distickstoffpentoxid, Salze von Metallen höherer Wertigkeit, wie Blei-, Wismut-, Vanadin-, Mangan-, Chrom-, Kobaltsalze. Auch die anodische Oxidation ist möglich. Die Oxidation kann nicht nur auf der Endstufe durchgeführt werden, sondern ist prinzipiell auf jeder Stufe des vorstehend beschriebenen Syntheseweges möglich.

Die folgenden Beispiele erläutern die Herstellung der neuen Verbindungen. Dabei verhalten sich Gewichtsteile zu Volumenteilen wie Kilogramm zu Liter.

Beispiel 1

14,8 Gew.-Teile 2-Propionyl-5-(5,6-dihydro-2H-pyran-3-yl)-3-hydroxy-2-cyclohexen-1-on werden in 100 Volumenteilen Dichlormethan gelöst und bei Raumtemperatur m-Chlorperbenzoesäure zugegeben, wobei die Umsetzung mittels Dünnschichtchromatographie verfolgt wird. Nach vollständiger Umsetzung wird die ausgefallene m-Chlorbenzoesäure abfiltriert, das Filtrat zweimal mit halbkonzentrierter Natriumhydrogencarbonatlösung und Wasser gewaschen, über Natriumsulfat getrocknet und das Lösungsmittel im Vakuum abdestilliert. Man erhält 2-Propionyl-5-(3,4-epoxy-tetrahydropyran-3-yl)-3-hydroxy-2-cyclohexen-1-on. (Verbindung Nr. 30)

Beispiel 2

3,0 Gew.-Teile 2-Propionyl-5-(3,4-epoxytetrahydropyran-3-yl)-3-hydroxy-2-cyclohexen-1-on, 1,8 Gew.-Teile (E)-3-Chlor-2-propenyloxyammoniumchlorid, 1,1 Gew.-Teile Natriumhydrogencarbonat und 100 Volumenteile Methanol werden bei Raumtemperatur 16 Stunden gerührt. Das Lösungsmittel wird unter vermindertem Druck abdestilliert, zum verbleibenden Rückstand werden jeweils 50 Volumenteile Wasser und Dichlormethan gegeben, nach heftigem Rühren die Phasen getrennt und die organische Phase über Natriumsulfat getrocknet. Nach Abdestillieren des Lösungsmittels unter vermindertem Druck erhält man 2-[1-((E)-3-Chlor-2-propenyloxyimino)propyl]-5-(3,4-epoxytetrahydropyran-3-yl)-3-hydroxy-2-cyclohexen-1-on. (Verbindung Nr. 401)

Die folgenden Verbindungen können in analoger Weise erhalten werden:

(Die $^1H$-NMR-Daten sind in δ (ppm) angegeben und auf Tetramethylsilan als internem Standard bezogen. Abkürzungen: s = Singulett, d = Dublett, m = Multiplett, q = Quartett).

**Tabelle 1**

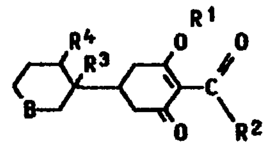

| Nr. | $R^1$ | $R^2$ | $R^3$ | $R^4$ | B | $^1$H-NMR-Daten |
|---|---|---|---|---|---|---|
| 1 | H | Methyl | Cl | Cl | S | |
| 2 | H | Methyl | Cl | Cl | O | |
| 3 | H | Methyl | Cl | Cl | SO | |
| 4 | H | Methyl | Cl | Cl | $SO_2$ | |
| 5 | H | Methyl | Br | Br | S | |
| 6 | H | Methyl | Br | Br | O | |
| 7 | H | Methyl | Br | Br | SO | |
| 8 | H | Methyl | Br | Br | $SO_2$ | |
| 9 | H | Methyl | OH | OH | S | |
| 10 | H | Methyl | OH | OH | O | |
| 11 | H | Methyl | OH | OH | SO | |
| 12 | H | Methyl | OH | OH | $SO_2$ | |
| 13 | H | Methyl | –O– | | S | |
| 14 | H | Methyl | –O– | | O | |
| 15 | H | Methyl | –O– | | SO | |
| 16 | H | Methyl | –O– | | $SO_2$ | |
| 17 | H | Ethyl | Cl | Cl | S | |
| 18 | H | Ethyl | Cl | Cl | O | |
| 19 | H | Ethyl | Cl | Cl | SO | |
| 20 | H | Ethyl | Cl | Cl | $SO_2$ | |
| 21 | H | Ethyl | Br | Br | S | |
| 22 | H | Ethyl | Br | Br | O | |

**Tabelle 1** Fortsetzung

| Nr. | $R^1$ | $R^2$ | $R^3$ | $R^4$ | B | $^1H$-NMR-Daten |
|---|---|---|---|---|---|---|
| 23 | H | Ethyl | Br | Br | SO | |
| 24 | H | Ethyl | Br | Br | $SO_2$ | |
| 25 | H | Ethyl | OH | OH | S | |
| 26 | H | Ethyl | OH | OH | O | |
| 27 | H | Ethyl | OH | OH | SO | |
| 28 | H | Ethyl | OH | OH | $SO_2$ | |
| 29 | H | Ethyl | –O– | | S | |
| 30 | H | Ethyl | –O– | | O | |
| 31 | H | Ethyl | –O– | | SO | |
| 32 | H | Ethyl | –O– | | $SO_2$ | |
| 33 | H | n-Propyl | Cl | Cl | S | |
| 34 | H | n-Propyl | Cl | Cl | O | 1,0(t), 3,9(m), 4,4(m) |
| 35 | H | n-Propyl | Cl | Cl | SO | |
| 36 | H | n-Propyl | Cl | Cl | $SO_2$ | |
| 37 | H | n-Propyl | Br | Br | S | |
| 38 | H | n-Propyl | Br | Br | O | 2,0(d), 3,0(m), 4,6(s) |
| 39 | H | n-Propyl | Br | Br | SO | |
| 40 | H | n-Propyl | Br | Br | $SO_2$ | |
| 41 | H | n-Propyl | OH | OH | S | |
| 42 | H | n-Propyl | OH | OH | O | 1,0(t), 1,6(q), 3,8(m) |
| 43 | H | n-Propyl | OH | OH | SO | |
| 44 | H | n-Propyl | OH | OH | $SO_2$ | |
| 45 | H | n-Propyl | –O– | | S | |
| 46 | H | n-Propyl | –O– | | O | |
| 47 | H | n-Propyl | –O– | | SO | |
| 48 | H | n-Propyl | –O– | | $SO_2$ | |

Tabelle 1 Fortsetzung

| Nr. | $R^1$ | $R^2$ | $R^3$ | $R^4$ | B | $^1$H-NMR-Daten |
|---|---|---|---|---|---|---|
| 49 | H | n-Butyl | Cl | Cl | S | |
| 50 | H | n-Butyl | Cl | Cl | O | |
| 51 | H | n-Butyl | Cl | Cl | SO | |
| 52 | H | n-Butyl | Cl | Cl | $SO_2$ | |
| 53 | H | n-Butyl | Br | Br | S | |
| 54 | H | n-Butyl | Br | Br | O | |
| 55 | H | n-Butyl | Br | Br | SO | |
| 56 | H | n-Butyl | Br | Br | $SO_2$ | |
| 57 | H | n-Butyl | OH | OH | S | |
| 58 | H | n-Butyl | OH | OH | O | |
| 59 | H | n-Butyl | OH | OH | SO | |
| 60 | H | n-Butyl | OH | OH | $SO_2$ | |
| 61 | H | n-Butyl | -O- | | S | |
| 62 | H | n-Butyl | -O- | | O | |
| 63 | H | n-Butyl | -O- | | SO | |
| 64 | H | n-Butyl | -O- | | $SO_2$ | |
| 65 | $Na^+$ | Methyl | Cl | Cl | S | |
| 66 | $Na^+$ | Methyl | Cl | Cl | O | |
| 67 | $Na^+$ | Methyl | Cl | Cl | SO | |
| 68 | $Na^+$ | Methyl | Cl | Cl | $SO_2$ | |
| 69 | $Na^+$ | Methyl | Br | Br | S | |
| 70 | $Na^+$ | Methyl | Br | Br | O | |
| 71 | $Na^+$ | Methyl | Br | Br | SO | |
| 72 | $Na^+$ | Methyl | Br | Br | $SO_2$ | |
| 73 | $Na^+$ | Methyl | OH | OH | S | |
| 74 | $Na^+$ | Methyl | OH | OH | O | |

**Tabelle 1** Fortsetzung

| Nr. | $R^1$ | $R^2$ | $R^3$ | $R^4$ | B | $^1H$-NMR-Daten |
|---|---|---|---|---|---|---|
| 75 | $Na^+$ | Methyl | OH | OH | SO | |
| 76 | $Na^+$ | Methyl | OH | OH | $SO_2$ | |
| 77 | $Na^+$ | Methyl | –O– | | S | |
| 78 | $Na^+$ | Methyl | –O– | | O | |
| 79 | $Na^+$ | Methyl | –O– | | SO | |
| 80 | $Na^+$ | Methyl | –O– | | $SO_2$ | |
| 81 | $Na^+$ | Ethyl | Cl | Cl | S | |
| 82 | $Na^+$ | Ethyl | Cl | Cl | O | |
| 83 | $Na^+$ | Ethyl | Cl | Cl | SO | |
| 84 | $Na^+$ | Ethyl | Cl | Cl | $SO_2$ | |
| 85 | $Na^+$ | Ethyl | Br | Br | S | |
| 86 | $Na^+$ | Ethyl | Br | Br | O | |
| 87 | $Na^+$ | Ethyl | Br | Br | SO | |
| 88 | $Na^+$ | Ethyl | Br | Br | $SO_2$ | |
| 89 | $Na^+$ | Ethyl | OH | OH | S | |
| 90 | $Na^+$ | Ethyl | OH | OH | O | |
| 91 | $Na^+$ | Ethyl | OH | OH | SO | |
| 92 | $Na^+$ | Ethyl | OH | OH | $SO_2$ | |
| 93 | $Na^+$ | Ethyl | –O– | | S | |
| 94 | $Na^+$ | Ethyl | –O– | | O | |
| 95 | $Na^+$ | Ethyl | –O– | | SO | |
| 96 | $Na^+$ | Ethyl | –O– | | $SO_2$ | |
| 97 | $Na^+$ | n-Propyl | Cl | Cl | S | |
| 98 | $Na^+$ | n-Propyl | Cl | Cl | O | |
| 99 | $Na^+$ | n-Propyl | Cl | Cl | SO | |
| 100 | $Na^+$ | n-Propyl | Cl | Cl | $SO_2$ | |

**Tabelle 1** Fortsetzung

| Nr. | $R^1$ | $R^2$ | $R^3$ | $R^4$ | B | $^1$H-NMR-Daten |
|---|---|---|---|---|---|---|
| 101 | $Na^+$ | n-Propyl | Br | Br | S | |
| 102 | $Na^+$ | n-Propyl | Br | Br | O | |
| 103 | $Na^+$ | n-Propyl | Br | Br | SO | |
| 104 | $Na^+$ | n-Propyl | Br | Br | $SO_2$ | |
| 105 | $Na^+$ | n-Propyl | OH | OH | S | |
| 106 | $Na^+$ | n-Propyl | OH | OH | O | |
| 107 | $Na^+$ | n-Propyl | OH | OH | SO | |
| 108 | $Na^+$ | n-Propyl | OH | OH | $SO_2$ | |
| 109 | $Na^+$ | n-Propyl | –O– | | S | |
| 110 | $Na^+$ | n-Propyl | –O– | | O | |
| 111 | $Na^+$ | n-Propyl | –O– | | SO | |
| 112 | $Na^+$ | n-Propyl | –O– | | $SO_2$ | |
| 113 | $Na^+$ | n-Butyl | Cl | Cl | S | |
| 114 | $Na^+$ | n-Butyl | Cl | Cl | O | |
| 115 | $Na^+$ | n-Butyl | Cl | Cl | SO | |
| 116 | $Na^+$ | n-Butyl | Cl | Cl | $SO_2$ | |
| 117 | $Na^+$ | n-Butyl | Br | Br | S | |
| 118 | $Na^+$ | n-Butyl | Br | Br | O | |
| 119 | $Na^+$ | n-Butyl | Br | Br | SO | |
| 120 | $Na^+$ | n-Butyl | Br | Br | $SO_2$ | |
| 121 | $Na^+$ | n-Butyl | OH | OH | S | |
| 122 | $Na^+$ | n-Butyl | OH | OH | O | |
| 123 | $Na^+$ | n-Butyl | OH | OH | SO | |
| 124 | $Na^+$ | n-Butyl | OH | OH | $SO_2$ | |
| 125 | $Na^+$ | n-Butyl | –O– | | S | |
| 126 | $Na^+$ | n-Butyl | –O– | | O | |

**Tabelle 1** Fortsetzung

| Nr. | $R^1$ | $R^2$ | $R^3$ | $R^4$ | B | $^1$H-NMR-Daten |
|---|---|---|---|---|---|---|
| 127 | $Na^+$ | n-Butyl | —O— | | SO | |
| 128 | $Na^+$ | n-Butyl | —O— | | $SO_2$ | |
| 129 | $K^+$ | Methyl | Cl | Cl | S | |
| 130 | $K^+$ | Methyl | Cl | Cl | O | |
| 131 | $K^+$ | Methyl | Cl | Cl | SO | |
| 132 | $K^+$ | Methyl | Cl | Cl | $SO_2$ | |
| 133 | $K^+$ | Methyl | Br | Br | S | |
| 134 | $K^+$ | Methyl | Br | Br | O | |
| 135 | $K^+$ | Methyl | Br | Br | SO | |
| 136 | $K^+$ | Methyl | Br | Br | $SO_2$ | |
| 137 | $K^+$ | Methyl | OH | OH | S | |
| 138 | $K^+$ | Methyl | OH | OH | O | |
| 139 | $K^+$ | Methyl | OH | OH | SO | |
| 140 | $K^+$ | Methyl | OH | OH | $SO_2$ | |
| 141 | $K^+$ | Methyl | —O— | | S | |
| 142 | $K^+$ | Methyl | —O— | | O | |
| 143 | $K^+$ | Methyl | —O— | | SO | |
| 144 | $K^+$ | Ethyl | —O— | | $SO_2$ | |
| 145 | $K^+$ | Ethyl | Cl | Cl | S | |
| 146 | $K^+$ | Ethyl | Cl | Cl | O | |
| 147 | $K^+$ | Ethyl | Cl | Cl | SO | |
| 148 | $K^+$ | Ethyl | Cl | Cl | $SO_2$ | |
| 149 | $K^+$ | Ethyl | Br | Br | S | |
| 150 | $K^+$ | Ethyl | Br | Br | O | |
| 151 | $K^+$ | Ethyl | Br | Br | SO | |
| 152 | $K^+$ | Ethyl | Br | Br | $SO_2$ | |

**Tabelle 1** Fortsetzung

| Nr. | $R^1$ | $R^2$ | $R^3$ | $R^4$ | B | $^1H$-NMR-Daten |
|---|---|---|---|---|---|---|
| 153 | $K^+$ | Ethyl | OH | OH | S | |
| 154 | $K^+$ | Ethyl | OH | OH | O | |
| 155 | $K^+$ | Ethyl | OH | OH | SO | |
| 156 | $K^+$ | Ethyl | OH | OH | $SO_2$ | |
| 157 | $K^+$ | Ethyl | –O– | | S | |
| 158 | $K^+$ | Ethyl | –O– | | O | |
| 159 | $K^+$ | Ethyl | –O– | | SO | |
| 160 | $K^+$ | Ethyl | –O– | | $SO_2$ | |
| 161 | $K^+$ | n-Propyl | Cl | Cl | S | |
| 162 | $K^+$ | n-Propyl | Cl | Cl | O | |
| 163 | $K^+$ | n-Propyl | Cl | Cl | SO | |
| 164 | $K^+$ | n-Propyl | Cl | Cl | $SO_2$ | |
| 165 | $K^+$ | n-Propyl | Br | Br | S | |
| 166 | $K^+$ | n-Propyl | Br | Br | O | |
| 167 | $K^+$ | n-Propyl | Br | Br | SO | |
| 168 | $K^+$ | n-Propyl | Br | Br | $SO_2$ | |
| 169 | $K^+$ | n-Propyl | OH | OH | S | |
| 170 | $K^+$ | n-Propyl | OH | OH | O | |
| 171 | $K^+$ | n-Propyl | OH | OH | SO | |
| 172 | $K^+$ | n-Propyl | OH | OH | $SO_2$ | |
| 173 | $K^+$ | n-Propyl | –O– | | S | |
| 174 | $K^+$ | n-Propyl | –O– | | O | |
| 175 | $K^+$ | n-Propyl | –O– | | SO | |
| 176 | $K^+$ | n-Propyl | –O– | | $SO_2$ | |
| 177 | $K^+$ | n-Butyl | Cl | Cl | S | |
| 178 | $K^+$ | n-Butyl | Cl | Cl | O | |

Tabelle 1 Fortsetzung

| Nr. | $R^1$ | $R^2$ | $R^3$ ... | $R^4$ | B | $^1H$-NMR-Daten |
|---|---|---|---|---|---|---|
| 179 | $K^+$ | n-Butyl | Cl | Cl | SO | |
| 180 | $K^+$ | n-Butyl | Cl | Cl | $SO_2$ | |
| 181 | $K^+$ | n-Butyl | Br | Br | S | |
| 182 | $K^+$ | n-Butyl | Br | Br | O | |
| 183 | $K^+$ | n-Butyl | Br | Br | SO | |
| 184 | $K^+$ | n-Butyl | Br | Br | $SO_2$ | |
| 185 | $K^+$ | n-Butyl | OH | OH | S | |
| 186 | $K^+$ | n-Butyl | OH | OH | O | |
| 187 | $K^+$ | n-Butyl | OH | OH | SO | |
| 188 | $K^+$ | n-Butyl | OH | OH | $SO_2$ | |
| 189 | $K^+$ | n-Butyl | —O— | | S | |
| 190 | $K^+$ | n-Butyl | —O— | | O | |
| 191 | $K^+$ | n-Butyl | —O— | | SO | |
| 192 | $K^+$ | n-Butyl | —O— | | $SO_2$ | |

Tabelle 2

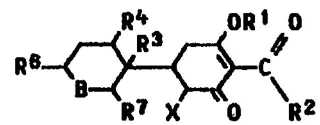

| Nr. | $R^1$ | $R^2$ | $R^3$ | $R^4$ | $R^6$ | $R^7$ | B | X | $^1$H-NMR-Daten |
|---|---|---|---|---|---|---|---|---|---|
| 193 | H | Ethyl | –O– | | H | H | O | $COOCH_3$ | |
| 194 | H | Ethyl | Cl | Cl | H | H | O | $COOCH_3$ | |
| 195 | Trimethylbenzylammonium | n-Propyl | –O– | | H | H | O | H | |
| 196 | H | n-Propyl | –O– | | Methyl | Methyl | O | H | |
| 197 | H | n-Propyl | Cl | Cl | H | H | O | $COOCH_3$ | |
| 198 | H | n-Propyl | Br | Br | H | H | O | $COOCH_3$ | |

**Tabelle 3**

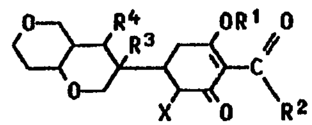

| Nr. | $R^1$ | $R^2$ | $R^3$ | $R^4$ | X | $^1$H-NMR-Daten |
|---|---|---|---|---|---|---|
| 199 | H | Methyl | Cl | Cl | H | |
| 200 | H | Methyl | Br | Br | H | |
| 201 | H | Methyl | OH | OH | H | |
| 202 | H | Methyl | –O– | | H | |
| 203 | H | Ethyl | Cl | Cl | H | |
| 204 | H | Ethyl | Br | Br | H | |
| 205 | H | Ethyl | OH | OH | H | |
| 206 | H | Ethyl | –O– | | H | |
| 207 | H | n-Propyl | Cl | Cl | H | |
| 208 | H | n-Propyl | Br | Br | H | 1,0(t), 1,7(q), 4,0(s) |
| 209 | H | n-Propyl | OH | OH | H | |
| 210 | H | n-Propyl | –O– | | H | 1,0(t), 2,5(m), 4,1(m) |
| 211 | H | n-Butyl | Cl | Cl | H | |
| 212 | H | n-Butyl | Br | Br | H | |
| 213 | H | n-Butyl | OH | OH | H | |
| 214 | H | n-Butyl | –O– | | H | |
| 215 | $Na^+$ | Methyl | Cl | Cl | H | |
| 216 | $Na^+$ | Methly | Br | Br | H | |
| 217 | $Na^+$ | Methyl | OH | OH | H | |
| 218 | $Na^+$ | Methyl | –O– | | H | |

**Tabelle 3** Fortsetzung

| Nr. | $R^1$ | $R^2$ | $R^3$ | $R^4$ | X | $^1H$-NMR-Daten |
|---|---|---|---|---|---|---|
| 219 | $Na^+$ | Ethyl | Cl | Cl | H | |
| 220 | $Na^+$ | Ethyl | Br | Br | H | |
| 221 | $Na^+$ | Ethyl | OH | OH | H | |
| 222 | $Na^+$ | Ethyl | –O– | | H | |
| 223 | $Na^+$ | n-Propyl | Cl | Cl | H | |
| 224 | $Na^+$ | n-Propyl | Br | Br | H | |
| 225 | $Na^+$ | n-Propyl | OH | OH | H | |
| 226 | $Na^+$ | n-Propyl | –O– | | H | |
| 227 | $Na^+$ | n-Butyl | Cl | Cl | H | |
| 228 | $Na^+$ | n-Butyl | Br | Br | H | |
| 229 | $Na^+$ | n-Butyl | OH | OH | H | |
| 230 | $Na^+$ | n-Butyl | –O– | | H | |
| 231 | $K^+$ | Methyl | Cl | Cl | H | |
| 232 | $K^+$ | Methyl | Br | Br | H | |
| 233 | $K^+$ | Methyl | OH | OH | H | |
| 234 | $K^+$ | Methyl | –O– | | H | |
| 235 | $K^+$ | Ethyl | Cl | Cl | H | |
| 236 | $K^+$ | Ethyl | Br | Br | H | |
| 237 | $K^+$ | Ethyl | OH | OH | H | |
| 238 | $K^+$ | Ethyl | –O– | | H | |
| 239 | $K^+$ | n-Propyl | Cl | Cl | H | |
| 240 | $K^+$ | n-Propyl | Br | Br | H | |
| 241 | $K^+$ | n-Propyl | OH | OH | H | |
| 242 | $K^+$ | n-Propyl | –O– | | H | |

**Tabelle 3** Fortsetzung

| Nr. | $R^1$ | $R^2$ | $R^3$ | $R^4$ | X | $^1$H-NMR-Daten |
|---|---|---|---|---|---|---|
| 243 | $K^+$ | n-Butyl | Cl | Cl | H | |
| 244 | $K^+$ | n-Butyl | Br | Br | H | |
| 245 | $K^+$ | n-Butyl | OH | OH | H | |
| 246 | $K^+$ | n-Butyl | –O– | | H | |
| 247 | H | n-Propyl | Cl | Cl | $COOCH_3$ | |
| 248 | H | Ethyl | Cl | Cl | $COOCH_3$ | |
| 249 | Trimethylbenzylammonium | Ethyl | Cl | Cl | H | |
| 250 | Trimethylbenzylammonium | n-Propyl | Cl | Cl | H | |
| 251 | Tetramethylammonium | Ethyl | –O– | | H | |
| 252 | Tetramethylammonium | Ethyl | Cl | Cl | $COOCH_3$ | |

**Tabelle 4**

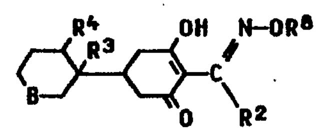

| Nr. | $R^2$ | $R^3$ | $R^4$ | B | $R^8$ | $^1$H-NMR-Daten |
|---|---|---|---|---|---|---|
| 253 | Methyl | Cl | Cl | S | Ethyl | |
| 254 | Methyl | Cl | Cl | S | Allyl | |
| 255 | Methyl | Cl | Cl | S | (E)-2-Butenyl | |
| 256 | Methyl | Cl | Cl | S | (E)-3-Chlor-2-propenyl | |
| 257 | Methyl | Cl | Cl | S | Propargyl | |
| 258 | Methyl | Cl | Cl | O | Ethyl | |
| 259 | Methyl | Cl | Cl | O | Allyl | |
| 260 | Methyl | Cl | Cl | O | (E)-2-Butenyl | |
| 261 | Methyl | Cl | Cl | O | (E)-3-Chlor-2-propenyl | |
| 262 | Methyl | Cl | Cl | O | Propargyl | |
| 263 | Methyl | Cl | Cl | SO | Ethyl | |
| 264 | Methyl | Cl | Cl | SO | Allyl | |
| 265 | Methyl | Cl | Cl | SO | (E)-2-Butenyl | |
| 266 | Methyl | Cl | Cl | SO | (E)-3-Chlor-2-propenyl | |
| 267 | Methyl | Cl | Cl | SO | Propargyl | |
| 268 | Methyl | Cl | Cl | $SO_2$ | Ethyl | |
| 269 | Methyl | Cl | Cl | $SO_2$ | Allyl | |
| 270 | Methyl | Cl | Cl | $SO_2$ | (E)-2-Butenyl | |
| 271 | Methyl | Cl | Cl | $SO_2$ | (E)-3-Chlor-2-propenyl | |
| 272 | Methyl | Cl | Cl | $SO_2$ | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 273 | Methyl | Br | Br | S | Ethyl | |
| 274 | Methyl | Br | Br | S | Allyl | |
| 275 | Methyl | Br | Br | S | (E)-2-Butenyl | |
| 276 | Methyl | Br | Br | S | (E)-3-Chlor-2-propenyl | |
| 277 | Methyl | Br | Br | S | Propargyl | |
| 278 | Methyl | Br | Br | O | Ethyl | |
| 279 | Methyl | Br | Br | O | Allyl | |
| 280 | Methyl | Br | Br | O | (E)-2-Butenyl | |
| 281 | Methyl | Br | Br | O | (E)-3-Chlor-2-propenyl | |
| 282 | Methyl | Br | Br | O | Propargyl | |
| 283 | Methyl | Br | Br | SO | Ethyl | |
| 284 | Methyl | Br | Br | SO | Allyl | |
| 285 | Methyl | Br | Br | SO | (E)-2-Butenyl | |
| 286 | Methyl | Br | Br | SO | (E)-3-Chlor-2-propenyl | |
| 287 | Methyl | Br | Br | SO | Propargyl | |
| 288 | Methyl | Br | Br | $SO_2$ | Ethyl | |
| 289 | Methyl | Br | Br | $SO_2$ | Allyl | |
| 290 | Methyl | Br | Br | $SO_2$ | (E)-2-Butenyl | |
| 291 | Methyl | Br | Br | $SO_2$ | (E)-3-Chlor-2-propenyl | |
| 292 | Methyl | Br | Br | $SO_2$ | Propargyl | |
| 293 | Methyl | OH | OH | S | Ethyl | |
| 294 | Methyl | OH | OH | S | Allyl | |
| 295 | Methyl | OH | OH | S | (E)-2-Butenyl | |
| 296 | Methyl | OH | OH | S | (E)-3-Chlor-2-propenyl | |
| 297 | Methyl | OH | OH | S | Propargyl | |

**Tabelle 4** Fortsetzung

| Nr. | $R^2$ | $R^3$ | $R^4$ | B | $R^8$ | $^1$H-NMR-Daten |
|---|---|---|---|---|---|---|
| 298 | Methyl | OH | OH | O | Ethyl | |
| 299 | Methyl | OH | OH | O | Allyl | |
| 300 | Methyl | OH | OH | O | (E)-2-Butenyl | |
| 301 | Methyl | OH | OH | O | (E)-3-Chlor-2-propenyl | |
| 302 | Methyl | OH | OH | O | Propargyl | |
| 303 | Methyl | OH | OH | SO | Ethyl | |
| 304 | Methyl | OH | OH | SO | Allyl | |
| 305 | Methyl | OH | OH | SO | (E)-2-Butenyl | |
| 306 | Methyl | OH | OH | SO | (E)-3-Chlor-2-propenyl | |
| 307 | Methyl | OH | OH | SO | Propargyl | |
| 308 | Methyl | OH | OH | $SO_2$ | Ethyl | |
| 309 | Methyl | OH | OH | $SO_2$ | Allyl | |
| 310 | Methyl | OH | OH | $SO_2$ | (E)-2-Butenyl | |
| 311 | Methyl | OH | OH | $SO_2$ | (E)-3-Chlor-2-propenyl | |
| 312 | Methyl | OH | OH | $SO_2$ | Propargyl | |
| 313 | Methyl | –O– | | S | Ethyl | |
| 314 | Methyl | –O– | | S | Allyl | |
| 315 | Methyl | –O– | | S | (E)-2-Butenyl | |
| 316 | Methyl | –O– | | S | (E)-3-Chlor-2-propenyl | |
| 317 | Methyl | –O– | | S | Propargyl | |
| 318 | Methyl | –O– | | O | Ethyl | |
| 319 | Methyl | –O– | | O | Allyl | |
| 320 | Methyl | –O– | | O | (E)-2-Butenyl | |
| 321 | Methyl | –O– | | O | (E)-3-Chlor-2-propenyl | |
| 322 | Methyl | –O– | | O | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 323 | Methyl | –O– | | SO | Ethyl | |
| 324 | Methyl | –O– | | SO | Allyl | |
| 325 | Methyl | –O– | | SO | (E)-2-Butenyl | |
| 326 | Methyl | –O– | | SO | (E)-3-Chlor-2-propenyl | |
| 327 | Methyl | –O– | | SO | Propargyl | |
| 328 | Methyl | –O– | | $SO_2$ | Ethyl | |
| 329 | Methyl | –O– | | $SO_2$ | Allyl | |
| 330 | Methyl | –O– | | $SO_2$ | (E)-2-Butenyl | |
| 331 | Methyl | –O– | | $SO_2$ | (E)-3-Chlor-2-propenyl | |
| 332 | Methyl | –O– | | $SO_2$ | Propargyl | |
| 333 | Ethyl | Cl | Cl | S | Ethyl | |
| 334 | Ethyl | Cl | Cl | S | Allyl | |
| 335 | Ethyl | Cl | Cl | S | (E)-2-Butenyl | |
| 336 | Ethyl | Cl | Cl | S | (E)-3-Chlor-2-propenyl | |
| 337 | Ethyl | Cl | Cl | S | Propargyl | |
| 338 | Ethyl | Cl | Cl | O | Ethyl | |
| 339 | Ethyl | Cl | Cl | O | Allyl | |
| 340 | Ethyl | Cl | Cl | O | (E)-2-Butenyl | |
| 341 | Ethyl | Cl | Cl | O | (E)-3-Chlor-2-propenyl | |
| 342 | Ethyl | Cl | Cl | O | Propargyl | |
| 343 | Ethyl | Cl | Cl | SO | Ethyl | |
| 344 | Ethyl | Cl | Cl | SO | Allyl | |
| 345 | Ethyl | Cl | Cl | SO | (E)-2-Butenyl | |
| 346 | Ethyl | Cl | Cl | SO | (E)-3-Chlor-2-propenyl | |
| 347 | Ethyl | Cl | Cl | SO | Propargyl | |

Tabelle 4 Fortsetzung

| Nr. | $R^2$ | $R^3$ | $R^4$ | B | $R^8$ | $^1H$-NMR-Daten |
|---|---|---|---|---|---|---|
| 348 | Ethyl | Cl | Cl | $SO_2$ | Ethyl | |
| 349 | Ethyl | Cl | Cl | $SO_2$ | Allyl | |
| 350 | Ethyl | Cl | Cl | $SO_2$ | (E)-2-Butenyl | |
| 351 | Ethyl | Cl | Cl | $SO_2$ | (E)-3-Chlor-2-propenyl | |
| 352 | Ethyl | Cl | Cl | $SO_2$ | Propargyl | |
| 353 | Ethyl | Br | Br | S | Ethyl | |
| 354 | Ethyl | Br | Br | S | Allyl | |
| 355 | Ethyl | Br | Br | S | (E)-2-Butenyl | |
| 356 | Ethyl | Br | Br | S | (E)-3-Chlor-2-propenyl | |
| 357 | Ethyl | Br | Br | S | Propargyl | |
| 358 | Ethyl | Br | Br | O | Ethyl | |
| 359 | Ethyl | Br | Br | O | Allyl | |
| 360 | Ethyl | Br | Br | O | (E)-2-Butenyl | |
| 361 | Ethyl | Br | Br | O | (E)-3-Chlor-2-propenyl | |
| 362 | Ethyl | Br | Br | O | Propargyl | |
| 363 | Ethyl | Br | Br | SO | Ethyl | |
| 364 | Ethyl | Br | Br | SO | Allyl | |
| 365 | Ethyl | Br | Br | SO | (E)-2-Butenyl | |
| 366 | Ethyl | Br | Br | SO | (E)-3-Chlor-2-propenyl | |
| 367 | Ethyl | Br | Br | SO | Propargyl | |
| 368 | Ethyl | Br | Br | $SO_2$ | Ethyl | |
| 369 | Ethyl | Br | Br | $SO_2$ | Allyl | |
| 370 | Ethyl | Br | Br | $SO_2$ | (E)-2-Butenyl | |
| 371 | Ethyl | Br | Br | $SO_2$ | (E)-3-Chlor-2-propenyl | |
| 372 | Ethyl | Br | Br | $SO_2$ | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 373 | Ethyl | OH | OH | S | Ethyl | |
| 374 | Ethyl | OH | OH | S | Allyl | |
| 375 | Ethyl | OH | OH | S | (E)-2-Butenyl | |
| 376 | Ethyl | OH | OH | S | (E)-3-Chlor-2-propenyl | |
| 377 | Ethyl | OH | OH | S | Propargyl | |
| 378 | Ethyl | OH | OH | O | Ethyl | |
| 379 | Ethyl | OH | OH | O | Allyl | |
| 380 | Ethyl | OH | OH | O | (E)-2-Butenyl | |
| 381 | Ethyl | OH | OH | O | (E)-3-Chlor-2-propenyl | |
| 382 | Ethyl | OH | OH | O | Propargyl | |
| 383 | Ethyl | OH | OH | SO | Ethyl | |
| 384 | Ethyl | OH | OH | SO | Allyl | |
| 385 | Ethyl | OH | OH | SO | (E)-2-Butenyl | |
| 386 | Ethyl | OH | OH | SO | (E)-3-Chlor-2-propenyl | |
| 387 | Ethyl | OH | OH | SO | Propargyl | |
| 388 | Ethyl | OH | OH | $SO_2$ | Ethyl | |
| 389 | Ethyl | OH | OH | $SO_2$ | Allyl | |
| 390 | Ethyl | OH | OH | $SO_2$ | (E)-2-Butenyl | |
| 391 | Ethyl | OH | OH | $SO_2$ | (E)-3-Chlor-2-propenyl | |
| 392 | Ethyl | OH | OH | $SO_2$ | Propargyl | |
| 393 | Ethyl | —O— | | S | Ethyl | |
| 394 | Ethyl | —O— | | S | Allyl | |
| 395 | Ethyl | —O— | | S | (E)-2-Butenyl | |
| 396 | Ethyl | —O— | | S | (E)-3-Chlor-2-propenyl | |
| 397 | Ethyl | —O— | | S | Propargyl | |

**Tabelle 4** Fortsetzung

| Nr. | $R^2$ | $R^3$ | $R^4$ | B | $R^8$ | $^1$H-NMR-Daten |
|---|---|---|---|---|---|---|
| 398 | Ethyl | –O– | | O | Ethyl | 1,3(t), 2,5(m), 3,3(s) |
| 399 | Ethyl | –O– | | O | Allyl | 1,1(t), 3,5(m), 4,6(d) |
| 400 | Ethyl | –O– | | O | (E)-2-Butenyl | |
| 401 | Ethyl | –O– | | O | (E)-3-Chlor-2-propenyl | 2,0(m), 3,5(m), 6,35(d) |
| 402 | Ethyl | –O– | | O | Propargyl | |
| 403 | Ethyl | –O– | | SO | Ethyl | |
| 404 | Ethyl | –O– | | SO | Allyl | |
| 405 | Ethyl | –O– | | SO | (E)-2-Butenyl | |
| 406 | Ethyl | –O– | | SO | (E)-3-Chlor-2-propenyl | |
| 407 | Ethyl | –O– | | SO | Propargyl | |
| 408 | Ethyl | –O– | | $SO_2$ | Ethyl | |
| 409 | Ethyl | –O– | | $SO_2$ | Allyl | |
| 410 | Ethyl | –O– | | $SO_2$ | (E)-2-Butenyl | |
| 411 | Ethyl | –O– | | $SO_2$ | (E)-3-Chlor-2-propenyl | |
| 412 | Ethyl | –O– | | $SO_2$ | Propargyl | |
| 413 | n-Propyl | Cl | Cl | S | Ethyl | |
| 414 | n-Propyl | Cl | Cl | S | Allyl | |
| 415 | n-Propyl | Cl | Cl | S | (E)-2-Butenyl | |
| 416 | n-Propyl | Cl | Cl | S | (E)-3-Chlor-2-propenyl | |
| 417 | n-Propyl | Cl | Cl | S | Propargyl | |
| 418 | n-Propyl | Cl | Cl | O | Ethyl | 1,3(t), 1,6(q), 4,4(s) |
| 419 | n-Propyl | Cl | Cl | O | Allyl | |
| 420 | n-Propyl | Cl | Cl | O | (E)-2-Butenyl | |
| 421 | n-Propyl | Cl | Cl | O | (E)-3-Chlor-2-propenyl | 1,0(t), 3,9(m), 4,5(d) |
| 422 | n-Propyl | Cl | Cl | O | Propargyl | |

**Tabelle 4 Fortsetzung**

| Nr. | $R^2$ | $R^3$ | $R^4$ | B | $R^8$ | $^1$H-NMR-Daten |
|---|---|---|---|---|---|---|
| 423 | n-Propyl | Cl | Cl | SO | Ethyl | |
| 424 | n-Propyl | Cl | Cl | SO | Allyl | |
| 425 | n-Propyl | Cl | Cl | SO | (E)-2-Butenyl | |
| 426 | n-Propyl | Cl | Cl | SO | (E)-3-Chlor-2-propenyl | |
| 427 | n-Propyl | Cl | Cl | SO | Propargyl | |
| 428 | n-Propyl | Cl | Cl | $SO_2$ | Ethyl | |
| 429 | n-Propyl | Cl | Cl | $SO_2$ | Allyl | |
| 430 | n-Propyl | Cl | Cl | $SO_2$ | (E)-2-Butenyl | |
| 431 | n-Propyl | Cl | Cl | $SO_2$ | (E)-3-Chlor-2-propenyl | |
| 432 | n-Propyl | Cl | Cl | $SO_2$ | Propargyl | |
| 433 | n-Propyl | Br | Br | S | Ethyl | |
| 434 | n-Propyl | Br | Br | S | Allyl | |
| 435 | n-Propyl | Br | Br | S | (E)-2-Butenyl | |
| 436 | n-Propyl | Br | Br | S | (E)-3-Chlor-2-propenyl | |
| 437 | n-Propyl | Br | Br | S | Propargyl | |
| 438 | n-Propyl | Br | Br | O | Ethyl | 1,3(t), 3,9(m), 4,65(s) |
| 439 | n-Propyl | Br | Br | O | Allyl | 1,9(d), 4,5(d), 4,6(s) |
| 440 | n-Propyl | Br | Br | O | (E)-2-Butenyl | 0,95(t), 1,8(d), 4,6(s) |
| 441 | n-Propyl | Br | Br | O | (E)-3-Chlor-2-propenyl | 0,95(t), 1,5(q), 6,3(d) |
| 442 | n-Propyl | Br | Br | O | Propargyl | |
| 443 | n-Propyl | Br | Br | SO | Ethyl | |
| 444 | n-Propyl | Br | Br | SO | Allyl | |
| 445 | n-Propyl | Br | Br | SO | (E)-2-Butenyl | |
| 446 | n-Propyl | Br | Br | SO | (E)-3-Chlor-2-propenyl | |
| 447 | n-Propyl | Br | Br | SO | Propargyl | |

Tabelle 4 Fortsetzung

| Nr. | $R^2$ | $R^3$ | $R^4$ | B | $R^8$ | $^1H$-NMR-Daten |
|---|---|---|---|---|---|---|
| 448 | n-Propyl | Br | Br | $SO_2$ | Ethyl | |
| 449 | n-Propyl | Br | Br | $SO_2$ | Allyl | |
| 450 | n-Propyl | Br | Br | $SO_2$ | (E)-2-Butenyl | |
| 451 | n-Propyl | Br | Br | $SO_2$ | (E)-3-Chlor-2-propenyl | |
| 452 | n-Propyl | Br | Br | $SO_2$ | Propargyl | |
| 453 | n-Propyl | OH | OH | S | Ethyl | |
| 454 | n-Propyl | OH | OH | S | Allyl | |
| 455 | n-Propyl | OH | OH | S | (E)-2-Butenyl | |
| 456 | n-Propyl | OH | OH | S | (E)-3-Chlor-2-propenyl | |
| 457 | n-Propyl | OH | OH | S | Propargyl | |
| 458 | n-Propyl | OH | OH | O | Ethyl | 1,0(t), 2,6(m), 4,1(q) |
| 459 | n-Propyl | OH | OH | O | Allyl | |
| 460 | n-Propyl | OH | OH | O | (E)-2-Butenyl | |
| 461 | n-Propyl | OH | OH | O | (E)-3-Chlor-2-propenyl | |
| 462 | n-Propyl | OH | OH | O | Propargyl | |
| 463 | n-Propyl | OH | OH | SO | Ethyl | |
| 464 | n-Propyl | OH | OH | SO | Allyl | |
| 465 | n-Propyl | OH | OH | SO | (E)-2-Butenyl | |
| 466 | n-Propyl | OH | OH | SO | (E)-3-Chlor-2-propenyl | |
| 467 | n-Propyl | OH | OH | SO | Propargyl | |
| 468 | n-Propyl | OH | OH | $SO_2$ | Ethyl | |
| 469 | n-Propyl | OH | OH | $SO_2$ | Allyl | |
| 470 | n-Propyl | OH | OH | $SO_2$ | (E)-2-Butenyl | |
| 471 | n-Propyl | OH | OH | $SO_2$ | (E)-3-Chlor-2-propenyl | |
| 472 | n-Propyl | OH | OH | $SO_2$ | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 473 | n-Propyl | –O– | | S | Ethyl | |
| 474 | n-Propyl | –O– | | S | Allyl | |
| 475 | n-Propyl | –O– | | S | (E)-2-Butenyl | |
| 476 | n-Propyl | –O– | | S | (E)-3-Chlor-2-propenyl | |
| 477 | n-Propyl | –O– | | S | Propargyl | |
| 478 | n-Propyl | –O– | | O | Ethyl | 1,35(t), 2,0(m), 3,3(s) |
| 479 | n-Propyl | –O– | | O | Allyl | |
| 480 | n-Propyl | –O– | | O | (E)-2-Butenyl | 0,95(t), 1,5(m), 3,3(s) |
| 481 | n-Propyl | –O– | | O | (E)-3-Chlor-2-propenyl | |
| 482 | n-Propyl | –O– | | O | Propargyl | |
| 483 | n-Propyl | –O– | | SO | Ethyl | |
| 484 | n-Propyl | –O– | | SO | Allyl | |
| 485 | n-Propyl | –O– | | SO | (E)-2-Butenyl | |
| 486 | n-Propyl | –O– | | SO | (E)-3-Chlor-2-propenyl | |
| 487 | n-Propyl | –O– | | SO | Propargyl | |
| 488 | n-Propyl | –O– | | $SO_2$ | Ethyl | |
| 489 | n-Propyl | –O– | | $SO_2$ | Allyl | |
| 490 | n-Propyl | –O– | | $SO_2$ | (E)-2-Butenyl | |
| 491 | n-Propyl | –O– | | $SO_2$ | (E)-3-Chlor-2-propenyl | |
| 492 | n-Propyl | –O– | | $SO_2$ | Propargyl | |
| 493 | n-Butyl | Cl | Cl | S | Ethyl | |
| 494 | n-Butyl | Cl | Cl | S | Allyl | |
| 495 | n-Butyl | Cl | Cl | S | (E)-2-Butenyl | |
| 496 | n-Butyl | Cl | Cl | S | (E)-3-Chlor-2-propenyl | |
| 497 | n-Butyl | Cl | Cl | S | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 498 | n-Butyl | Cl | Cl | O | Ethyl | |
| 499 | n-Butyl | Cl | Cl | O | Allyl | |
| 500 | n-Butyl | Cl | Cl | O | (E)-2-Butenyl | |
| 501 | n-Butyl | Cl | Cl | O | (E)-3-Chlor-2-propenyl | |
| 502 | n-Butyl | Cl | Cl | O | Propargyl | |
| 503 | n-Butyl | Cl | Cl | SO | Ethyl | |
| 504 | n-Butyl | Cl | Cl | SO | Allyl | |
| 505 | n-Butyl | Cl | Cl | SO | (E)-2-Butenyl | |
| 506 | n-Butyl | Cl | Cl | SO | (E)-3-Chlor-2-propenyl | |
| 507 | n-Butyl | Cl | Cl | SO | Propargyl | |
| 508 | n-Butyl | Cl | Cl | $SO_2$ | Ethyl | |
| 509 | n-Butyl | Cl | Cl | $SO_2$ | Allyl | |
| 510 | n-Butyl | Cl | Cl | $SO_2$ | (E)-2-Butenyl | |
| 511 | n-Butyl | Cl | Cl | $SO_2$ | (E)-3-Chlor-2-propenyl | |
| 512 | n-Butyl | Cl | Cl | $SO_2$ | Propargyl | |
| 513 | n-Butyl | Br | Br | S | Ethyl | |
| 514 | n-Butyl | Br | Br | S | Allyl | |
| 515 | n-Butyl | Br | Br | S | (E)-2-Butenyl | |
| 516 | n-Butyl | Br | Br | S | (E)-3-Chlor-2-propenyl | |
| 517 | n-Butyl | Br | Br | S | Propargyl | |
| 518 | n-Butyl | Br | Br | O | Ethyl | |
| 519 | n-Butyl | Br | Br | O | Allyl | |
| 520 | n-Butyl | Br | Br | O | (E)-2-Butenyl | |
| 521 | n-Butyl | Br | Br | O | (E)-3-Chlor-2-propenyl | |
| 522 | n-Butyl | Br | Br | O | Propargyl | |

Tabelle 4 Fortsetzung

| Nr. | $R^2$ | $R^3$ | $R^4$ | B | $R^8$ | $^1$H-NMR-Daten |
|---|---|---|---|---|---|---|
| 523 | n-Butyl | Br | Br | SO | Ethyl | |
| 524 | n-Butyl | Br | Br | SO | Allyl | |
| 525 | n-Butyl | Br | Br | SO | (E)-2-Butenyl | |
| 526 | n-Butyl | Br | Br | SO | (E)-3-Chlor-2-propenyl | |
| 527 | n-Butyl | Br | Br | SO | Propargyl | |
| 528 | n-Butyl | Br | Br | $SO_2$ | Ethyl | |
| 529 | n-Butyl | Br | Br | $SO_2$ | Allyl | |
| 530 | n-Butyl | Br | Br | $SO_2$ | (E)-2-Butenyl | |
| 531 | n-Butyl | Br | Br | $SO_2$ | (E)-3-Chlor-2-propenyl | |
| 532 | n-Butyl | Br | Br | $SO_2$ | Propargyl | |
| 533 | n-Butyl | OH | OH | S | Ethyl | |
| 534 | n-Butyl | OH | OH | S | Allyl | |
| 535 | n-Butyl | OH | OH | S | (E)-2-Butenyl | |
| 536 | n-Butyl | OH | OH | S | (E)-3-Chlor-2-propenyl | |
| 537 | n-Butyl | OH | OH | S | Propargyl | |
| 538 | n-Butyl | OH | OH | O | Ethyl | |
| 539 | n-Butyl | OH | OH | O | Allyl | |
| 540 | n-Butyl | OH | OH | O | (E)-2-Butenyl | |
| 541 | n-Butyl | OH | OH | O | (E)-3-Chlor-2-propenyl | |
| 542 | n-Butyl | OH | OH | O | Propargyl | |
| 543 | n-Butyl | OH | OH | SO | Ethyl | |
| 544 | n-Butyl | OH | OH | SO | Allyl | |
| 545 | n-Butyl | OH | OH | SO | (E)-2-Butenyl | |
| 546 | n-Butyl | OH | OH | SO | (E)-3-Chlor-2-propenyl | |
| 547 | n-Butyl | OH | OH | SO | Propargyl | |

**Tabelle 4 Fortsetzung**

| Nr. | $R^2$ | $R^3$ | $R^4$ | B | $R^8$ | $^1$H-NMR-Daten |
|---|---|---|---|---|---|---|
| 548 | n-Butyl | OH | OH | $SO_2$ | Ethyl | |
| 549 | n-Butyl | OH | OH | $SO_2$ | Allyl | |
| 550 | n-Butyl | OH | OH | $SO_2$ | (E)-2-Butenyl | |
| 551 | n-Butyl | OH | OH | $SO_2$ | (E)-3-Chlor-2-propenyl | |
| 552 | n-Butyl | OH | OH | $SO_2$ | Propargyl | |
| 553 | n-Butyl | —O— | | S | Ethyl | |
| 554 | n-Butyl | —O— | | S | Allyl | |
| 555 | n-Butyl | —O— | | S | (E)-2-Butenyl | |
| 556 | n-Butyl | —O— | | S | (E)-3-Chlor-2-propenyl | |
| 557 | n-Butyl | —O— | | S | Propargyl | |
| 558 | n-Butyl | —O— | | O | Ethyl | |
| 559 | n-Butyl | —O— | | O | Allyl | |
| 560 | n-Butyl | —O— | | O | (E)-2-Butenyl | |
| 561 | n-Butyl | —O— | | O | (E)-3-Chlor-2-propenyl | |
| 562 | n-Butyl | —O— | | O | Propargyl | |
| 563 | n-Butyl | —O— | | SO | Ethyl | |
| 564 | n-Butyl | —O— | | SO | Allyl | |
| 565 | n-Butyl | —O— | | SO | (E)-2-Butenyl | |
| 566 | n-Butyl | —O— | | SO | (E)-3-Chlor-2-propenyl | |
| 567 | n-Butyl | —O— | | SO | Propargyl | |
| 568 | n-Butyl | —O— | | $SO_2$ | Ethyl | |
| 569 | n-Butyl | —O— | | $SO_2$ | Allyl | |
| 570 | n-Butyl | —O— | | $SO_2$ | (E)-2-Butenyl | |
| 571 | n-Butyl | —O— | | $SO_2$ | (E)-3-Chlor-2-propenyl | |
| 572 | n-Butyl | —O— | | $SO_2$ | Propargyl | |

Tabelle 5

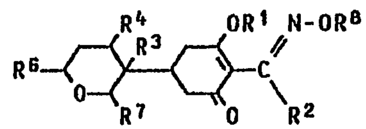

| Nr. | $R^1$ | $R^2$ | $R^3$ | $R^4$ | $R^6$ | $R^7$ | $R^8$ | $^1$H-NMR-Daten |
|---|---|---|---|---|---|---|---|---|
| 574 | H | n-Propyl | OH | Methylthio | H | H | Ethyl | |
| 575 | H | n-Propyl | OH | Ethylthio | H | H | Ethyl | |
| 576 | H | n-Propyl | OH | Methylcarbonylthio | H | H | Ethyl | |
| 577 | H | n-Propal | OH | Thiomethylcarboxyl | H | H | Ethyl | |
| 582 | Benzoyl | n-Propyl | OH | Methylthio | H | H | Ethyl | |
| 588 | Benzoyl | n-Propyl | –O– | | H | H | Ethyl | |
| 589 | Stearoyl | n-Propyl | –O– | | H | H | Ethyl | |
| 590 | H | n-Propyl | Methylthio | OH | Methyl | Methyl | Ethyl | |
| 591 | H | n-Propyl | Ethylthio | OH | Methyl | Methyl | Ethyl | |
| 672 | H | n-Propyl | –O– | | Methyl | Methyl | Ethyl | 0,96(t),3,91(q) 4,09(q) |

Tabelle 6

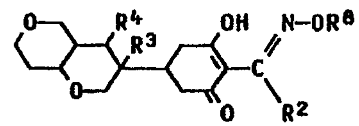

| Nr. | $R^2$ | $R^3$ | $R^4$ | $R^8$ | $^1$H-NMR-Daten |
|---|---|---|---|---|---|
| 592 | Methyl | Cl | Cl | Ethyl | |
| 593 | Methyl | Cl | Cl | Allyl | |
| 594 | Methyl | Cl | Cl | (E)-2-Butenyl | |
| 595 | Methyl | Cl | Cl | (E)-3-Chlor-2-propenyl | |
| 596 | Methyl | Cl | Cl | Propargyl | |
| 597 | Methyl | Br | Br | Ethyl | |
| 598 | Methyl | Br | Br | Allyl | |
| 599 | Methyl | Br | Br | (E)-2-Butenyl | |
| 600 | Methyl | Br | Br | (E)-3-Chlor-2-propenyl | |
| 601 | Methyl | Br | Br | Propargyl | |
| 602 | Methyl | OH | OH | Ethyl | |
| 603 | Methyl | OH | OH | Allyl | |
| 604 | Methyl | OH | OH | (E)-2-Butenyl | |
| 605 | Methyl | OH | OH | (E)-3-Chlor-2-propenyl | |
| 606 | Methyl | OH | OH | Propargyl | |
| 607 | Methyl | -O- | | Ethyl | |
| 608 | Methyl | -O- | | Allyl | |
| 609 | Methyl | -O- | | (E)-2-Butenyl | |
| 610 | Methyl | -O- | | (E)-3-Chlor-2-propenyl | |
| 611 | Methyl | -O- | | Propargyl | |

Tabelle 6 Fortsetzung

| Nr. | $R^2$ | $R^3$ | $R^4$ | $R^8$ | $^1$H-NMR-Daten |
|---|---|---|---|---|---|
| 612 | Ethyl | Cl | Cl | Ethyl | |
| 613 | Ethyl | Cl | Cl | Allyl | |
| 614 | Ethyl | Cl | Cl | (E)-2-Butenyl | |
| 615 | Ethyl | Cl | Cl | (E)-3-Chlor-2-propenyl | |
| 616 | Ethyl | Cl | Cl | Propargyl | |
| 617 | Ethyl | Br | Br | Ethyl | |
| 618 | Ethyl | Br | Br | Allyl | |
| 619 | Ethyl | Br | Br | (E)-2-Butenyl | |
| 620 | Ethyl | Br | Br | (E)-3-Chlor-2-propenyl | |
| 621 | Ethyl | Br | Br | Propargyl | |
| 622 | Ethyl | OH | OH | Ethyl | |
| 623 | Ethyl | OH | OH | Allyl | |
| 624 | Ethyl | OH | OH | (E)-2-Butenyl | |
| 625 | Ethyl | OH | OH | (E)-3-Chlor-2-propenyl | |
| 626 | Ethyl | OH | OH | Propargyl | |
| 627 | Ethyl | –O– | | Ethyl | |
| 628 | Ethyl | –O– | | Allyl | |
| 629 | Ethyl | –O– | | (E)-2-Butenyl | |
| 630 | Ethyl | –O– | | (E)-3-Chlor-2-propenyl | |
| 631 | Ethyl | –O– | | Propargyl | |
| 632 | n-Propyl | Cl | Cl | Ethyl | |
| 633 | n-Propyl | Cl | Cl | Allyl | |
| 634 | n-Propyl | Cl | Cl | (E)-2-Butenyl | |
| 635 | n-Propyl | Cl | Cl | (E)-3-Chlor-2-propenyl | |
| 636 | n-Propyl | Cl | Cl | Propargyl | |

Tabelle 6 Fortsetzung

| Nr. | $R^2$ | $R^3$ | $R^4$ | $R^8$ | $^1$H-NMR-Daten |
|---|---|---|---|---|---|
| 637 | n-Propyl | Br | Br | Ethyl | 1,6(q), 2,6(m), 4,1(q) |
| 638 | n-Propyl | Br | Br | Allyl | 1,0(t), 3,9(m), 5,4(m) |
| 639 | n-Propyl | Br | Br | (E)-2-Butenyl | 1,0(t), 1,7(d), 2,9(m) |
| 640 | n-Propyl | Br | Br | (E)-3-Chlor-2-propenyl | 1,5(q), 2,9(t), 4,0(s) |
| 641 | n-Propyl | Br | Br | Propargyl | |
| 642 | n-Propyl | OH | OH | Ethyl | |
| 643 | n-Propyl | OH | OH | Allyl | |
| 644 | n-Propyl | OH | OH | (E)-2-Butenyl | |
| 645 | n-Propyl | OH | OH | (E)-3-Chlor-2-propenyl | |
| 646 | n-Propyl | OH | OH | Propargyl | |
| 647 | n-Propyl | -O- | | Ethyl | 1,3(t), 2,3(m), 3,1(s) |
| 648 | n-Propyl | -O- | | Allyl | 0,95(t), 3,1(s), 4,5(d) |
| 649 | n-Propyl | -O- | | (E)-2-Butenyl | |
| 650 | n-Propyl | -O- | | (E)-3-Chlor-2-propenyl | 1,35(t), 2,0(m), 3,3(s) |
| 651 | n-Propyl | -O- | | Propargyl | |
| 652 | n-Butyl | Cl | Cl | Ethyl | |
| 653 | n-Butyl | Cl | Cl | Allyl | |
| 654 | n-Butyl | Cl | Cl | (E)-2-Butenyl | |
| 655 | n-Butyl | Cl | Cl | (E)-3-Chlor-2-propenyl | |
| 656 | n-Butyl | Cl | Cl | Propargyl | |
| 657 | n-Butyl | Br | Br | Ethyl | |
| 658 | n-Butyl | Br | Br | Allyl | |
| 659 | n-Butyl | Br | Br | (E)-2-Butenyl | |
| 660 | n-Butyl | Br | Br | (E)-3-Chlor-2-propenyl | |
| 661 | n-Butyl | Br | Br | Propargyl | |

Tabelle 6 Fortsetzung

| Nr. | $R^2$ | $R^3$ | $R^4$ | $R^8$ | $^1H$-NMR-Daten |
|---|---|---|---|---|---|
| 662 | n-Butyl | OH | OH | Ethyl | |
| 663 | n-Butyl | OH | OH | Allyl | |
| 664 | n-Butyl | OH | OH | (E)-2-Butenyl | |
| 665 | n-Butyl | OH | OH | (E)-3-Chlor-2-propenyl | |
| 666 | n-Butyl | OH | OH | Propargyl | |
| 667 | n-Butyl | -O- | | Ethyl | |
| 668 | n-Butyl | -O- | | Allyl | |
| 669 | n-Butyl | -O- | | (E)-2-Butenyl | |
| 670 | n-Butyl | -O- | | (E)-3-Chlor-2-propenyl | |
| 671 | n-Butyl | -O- | | Propargyl | |

Tabelle 7

| Nr. | A | B | X | $R^1$ | $R^2$ | $R^3$ | $R^4$ | $R^5$ | $R^6$ | $R^7$ | $R^8$ | |
|---|---|---|---|---|---|---|---|---|---|---|---|---|
| 673 | $NOR^8$ | O | H | $C_3H_7CO$ | Propyl | -O- | | $-CH_2OC_2H_4-$ | | H | Ethyl | 0,90(t);1,0(t);1,24(t) |
| 674 | $NOR^8$ | O | H | Benzoyl | Propyl | -O- | | $-CH_2OC_2H_4-$ | | H | Ethyl | 0,86(t);1,09(t);7,3-8,2(m) |
| 675 | $NOR^8$ | O | H | Acetyl | Propyl | -O- | | $-CH_2OC_2H_4-$ | | H | Ethyl | 0,88(t);1,25(t);2,19(s) |

Die Cyclohexenonderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittleren Naphthalinen, aromatische Kohlenwasserstoffe (Toluol, Xylol), Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 647 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 648 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 650 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 637 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 638 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 640 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 478 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 480 werden mit 2 Teilen Calciumsalz der Dodecylbenzolsulfonsäure, 8 Teilen Fettalkoholpolyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

IX. 10 Gew.-Teile des Wirkstoffs Nr. 440 werden mit 90 Gewichtsteilen einer Mischung aus 93 Gewichtsteilen Xylol und 7 Gewichtsteilen des Anlagerungsproduktes von 8 Mol Ethylenoxid an 1 Mol Nonylphenol gemischt. Man erhält auf diese Weise eine Lösung, die 10 Gew.% des Wirkstoffs enthält.

Die Applikation der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 3 kg/ha, vorzugsweise 0,05 bis 0,5 kg/ha.

Die Wirkung der Cyclohexenonderivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,125 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Alopecurus myosuroides | Ackerfuchsschwanz |
| Avena fatua | Flughafer |
| Avena sativa | Hafer |
| Digitaria sanguinalis | Blutfingerhirse |
| Echinochloa crus galli | Hühnerhirse |
| Lolium multiflorum | Ital. Raygras |
| Medicago sativa | Luzerne |
| Setaria italica | Kolbenhirse |
| Sinapis alba | Weißer Senf |
| Sorghum halepense | Wilde Mohrenhirse |
| Triticum aestivum | Weizen |
| Zea mays | Mais |

Mit den beispielhaft ausgewählten Verbindungen 647, 648, 650, 637, 638, 640, 478, 480, 440 lassen sich Gräser aus der Familie der Gramineen mit 3,0 kg Wirkstoff/ha im Vorauflaufverfahren gut bekämpfen. Sinapis alba (breitblättrige Pflanze) erleidet dabei keinerlei Schädigung.

Die Verbindungen 647, 648, 650 und 401 eignen sich mit 0,125 kg Wirkstoff/ha zur Bekämpfung eines breiten Spektrums unerwünschter grasartiger Pflanzen im Nachauflaufverfahren. Auch Ausfallkulturen aus der Familie der Gramineen werden erfaßt. Luzerne als breitblättrige Kulturpflanze bleibt hingegen völlig unbeeinflußt.

Unerwünschte Grasarten lassen sich mit den Wirkstoffen 438,439 und 674 im Nachauflaufverfahren bekämpfen, ohne die Kulturpflanze Weizen zu beeinträchtigen.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |

| Botanischer Name | Deutscher Name |
|---|---|
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |

| Botanischer Name | Deutscher Name |
|---|---|
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Cyclohexenonderivat der Formel

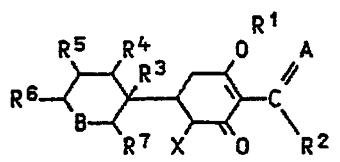

(I) ,

in der die Substituenten folgende Bedeutung haben:
A Sauerstoff oder ein Rest $NOR^8$, wobei $R^8$ eine $C_1$–$C_4$-Alkylgruppe, eine $C_3$–$C_4$-Alkenylgruppe, eine $C_3$–$C_4$-Alkinylgruppe, eine ein- bis dreifach durch Halogenatome substituierte $C_2$–$C_4$-Alkyl- oder $C_2$–$C_4$-Alkenylgruppe, eine $C_2$–$C_4$-Alkoxyalkylgruppe oder ein Chlorthienylrest bedeutet,
B ein Sauerstoff- oder Schwefelatom oder eine Sulfon- oder Sulfoxidgruppe,
X Wasserstoff oder eine Methoxycarbonylgruppe,
$R^1$ Wasserstoff, eine $C_2$–$C_{18}$-Alkylcarbonylgruppe, ein Benzoylrest oder ein anorganisches oder organisches Kation,
$R^2$ eine $C_1$–$C_4$-Alkylgruppe,
$R^3$ eine $C_1$–$C_3$-Alkoxygruppe oder eine $C_1$–$C_3$-Alkylthiogruppe, wenn $R^4$ eine Hydroxygruppe bedeutet,
$R^4$ eine $C_1$–$C_3$-Alkoxygruppe oder eine $C_1$–$C_3$-Alkylthiogruppe, wenn $R^3$ eine Hydroxygruppe bedeutet, oder
$R^3$, $R^4$ unabhängig voneinander Hydroxy, Chlor, Brom, $C_2$–$C_4$-Alkylcarbonylthio, $C_2$–$C_4$-Alkylcarbonyloxy oder gemeinsam ein Sauerstoffatom,
$R^5$, $R^6$ Wasserstoff, eine Methylgruppe oder gemeinsam eine Methylenoxyethylengruppe und
$R^7$ Wasserstoff oder eine Methylgruppe.

2. Cyclohexenonderivat gemäß Anspruch 1, dadurch gekennzeichnet, daß
A den Rest $NOR^8$,
B Sauerstoff,
X Wasserstoff,
$R^1$ Wasserstoff,
$R^2$ Ethyl oder Propyl,
$R^3$ und $R^4$ gleichzeitig Brom oder gemeinsam Epoxy,
$R^5$ und $R^6$ gleichzeitig Wasserstoff oder gmeinsam Methylenoxyethylen,
$R^7$ Wasserstoff und
$R^8$ Ethyl, Allyl, Chlorpropenyl oder Butenyl bedeuten.

3. Herbizid gemäß Anspruch 5, dadurch gekennzeichnet, daß
A den Rest $NOR^8$,
B Sauerstoff,
X Wasserstoff,
$R^1$ Wasserstoff,
$R^2$ Ethyl oder Propyl,
$R^3$ und $R^4$ gleichzeitig Brom oder gemeinsam Epoxy,
$R^5$ und $R^6$ gleichzeitig Wasserstoff oder gemeinsam Methylenoxyethylen,
$R^7$ Wasserstoff und
$R^8$ Ethyl, Allyl, Chlorpropenyl oder Butenyl bedeuten.

4. Verfahren zur Herstellung eines Cyclohexenonderivates der Formel I gemäß Anspruch 1, mit den Bedeutungen A = Sauerstoff und $R^1$ = Wasserstoff

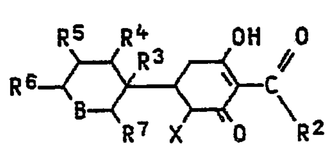

dadurch gekennzeichnet, daß man eine Verbindung der Formel II mit entsprechenden Resten gemäß Anspruch 1

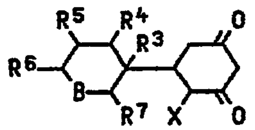
(II).

mit einem Säurechlorid $R^2COCl$ und einer Base in den Enolester überführt und diesen in Gegenwart von Imidazol- oder Pyridinderivaten umlagert.

5. Verfahren zur Herstellung eines Cyclohexenonderivates der Formel I gemäß Anspruch 1 mit den Bedeutungen A = $NOR^8$ und $R^1$ = Wasserstoff

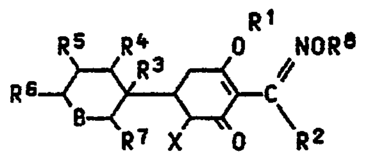

dadurch gekennzeichnet, daß man eine Verbindung der Formel I mit den Bedeutungen A = Sauerstoff und $R^1$ = Wasserstoff mit einem O-Aliphatenhydroxylamin $R^8ONH_2$ umsetzt, wobei $R^8$ die in Anspruch 1 angegebene Bedeutung hat.

6. Herbizid, enthaltend ein Cyclohexenonderivat der Formel I gemäß Anspruch 1 mit der Bedeutung A = $NOR^8$.

7. Herbizid, enthaltend einen inerten Zusatzstoff und ein Cyclohexenonderivat der Formel I gemäß Anspruch 1 mit der Bedeutung A = $NOR^8$.

8. Verfahren zur Bekämpfung unerwünschten Pflanzenwachstums, dadurch gekennzeichnet, daß man die unerwünschten Pflanzen oder die von unerwünschtem Pflanzenwachstum freizuhaltende Fläche mit einer herbizid wirksamen Menge eines Cyclohexenonderivats der Formel I gemäß Anspruch 1, in der A eine $NOR^8$-Gruppe bedeutet, behandelt.

## Claims

1. A cyclohexenone derivative of the formula I

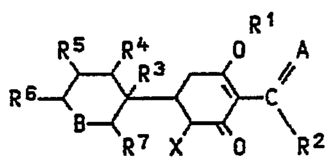
(I)

where
A is oxygen or $NOR^8$, where $R^8$ is $C_1$–$C_4$-alkyl, $C_1$–$C_4$-alkenyl, $C_3$–$C_4$-alkynyl, monohalogenated, dihalogenated or trihalogenated $C_2$–$C_4$-alkyl or $C_2$–$C_4$-alkenyl, $C_2$–$C_4$-alkoxyalkyl or chlorothienyl,
B is oxygen or sulfur or a sulfone or sulfoxide group,
X is hydrogen or methoxycarbonyl,
$R^1$ is hydrogen, $C_2$–$C_{18}$-alkylcarbonyl, benzoyl or an inorganic or organic cation,
$R^2$ is $C_1$–$C_4$-alkyl,
$R^3$ is $C_1$–$C_3$-alkoxy or $C_1$–$C_3$-alkylthio when $R^4$ is hydroxyl,
$R^4$ is $C_1$–$C_3$-alkoxy or $C_1$–$C_3$-alkylthio when $R^3$ is hydroxyl, or
$R^3$ and $R^4$ are each independently of the other hydroxyl, chlorine, bromine, $C_2$–$C_4$-alkylcarbonylthio, $C_2$–$C_4$-alkylcarbonyloxy or together oxygen,
$R^5$ and $R^6$ are each hydrogen, methyl or together methylene-oxyethylene, and
$R^7$ is hydrogen or methyl.

2. A cyclohexenone derivative as claimed in claim 1, wherein
A is $NOR^8$,
B is oxygen,
X is hydrogen,
$R^1$ is hydrogen,
$R^2$ is ethyl or propyl,
$R^3$ and $R^4$ are both bromine or together epoxy,
$R^5$ and $R^6$ are both hydrogen or together methyleneoxyethylene,
$R^7$ is hydrogen and
$R^8$ is ethyl, allyl, chloropropenyl or butenyl.

3. A herbicide as claimed in claim 1, wherein
A is $NOR^8$,

B is oxygen,
X is hydrogen,
$R^1$ is hydrogen,
$R^2$ is ethyl or propyl,
$R^3$ and $R^4$ are both bromine or together epoxy,
$R^5$ and $R^6$ are both hydrogen or together methyleneoxy-ethylene,
$R^7$ is hydrogen and
$R^8$ is ethyl, allyl, chloropropenyl or butenyl.

4. A process for preparing a cyclohexenone derivative of the formula I as claimed in claim 1 where A is oxygen and $R^1$ is hydrogen

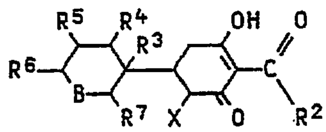

which comprises converting a compound of the formula II

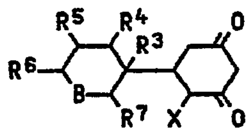

(II)

where the meanings are as defined in claim 1, with an acid chloride $R^2COCl$ and a base into the enol ester and rearranging said enol ester in the presence of an imidazole or pyridine derivative.

5. A process for preparing a cyclohexenone derivative of the formula I as claimed in claim 1 where A is $NOR^8$ and $R^1$ is hydrogen

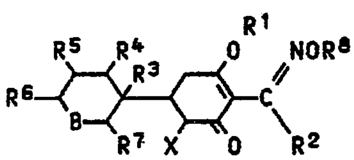

which comprises reacting a compound of the formula I where A is oxygen and $R^1$ is hydrogen with an O-aliphat-hydroxylamine $R^8NOH_2$, where $R^8$ is as defined in claim 1.

6. A herbicide containing a cyclohexenone derivative of the formula I as claimed in claim 1 where A is $NOR^8$.

7. A herbioide containing an inert additive and a cyclohexenone derivative of the formula I as claimed in claim 1 where A is $NOR^8$.

8. A process for controlling unwanted plant growth, which comprises treating the unwanted plants or the area to be kept free of unwanted plant growth with a herbicidally effective amount of a cyclohexenone derivative of the formula I as claimed in claim 1 where A is $NOR^8$.

## Revendications

1. Dérivé de cyclohéxénone de formule I

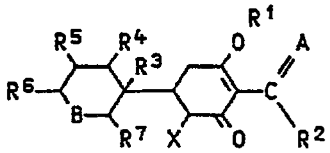

(I) ,

dans laquelle les substituants ont la signification suivante:
A est oxygène ou un reste $NOR^8$, $R^8$ représentant un groupe alkyle en $C_1$–$C_4$, un groupe alcényle en $C_3$–$C_4$, un groupe alcinyle en $C_3$–$C_4$, un groupe alkyle en $C_2$–$C_4$ ou alcényle en $C_2$–$C_4$ substitué une ou trois fois par des atomes d'halogène, un groupe alcoxyalkyle en $C_2$–$C_4$ ou un reste chlorothiényle,
B est un atome d'oxygène ou de soufre ou un groupe sulfon- ou sulfoxyde
X est hydrogène ou un groupe méthoxycarbonyle

$R^1$ est hydrogène, un groupe alkylcarbonyle en $C_2$–$C_{18}$, un reste benzoyle ou un cation inorganique ou organique.
$R^2$, un groupe alkyle en $C_1$–$C_4$,
$R^3$, un groupe alcoxy en $C_1$–$C_3$ ou un groupe alkylthio en $C_1$–$C_3$ quand $R^4$ représente un groupe hydroxy
$R^4$, un groupe alcoxy en $C_1$–$C_3$ ou un groupe alkyl (en $C_1$–$C_4$)-thio quand $R^3$ représente un groupe hydroxy, ou
$R^3$, $R^4$, indépendamment l'un de l'autre, hydroxy, chlore, brome, alkyle (en $C_1$–$C_4$) carbonylthio, alkyle (en $C_2$–$C_4$) carbonyloxy ou ensemble un atome d'oxygène
$R^5$, $R^6$ hydrogène, un groupe méthyle ou ensemble un groupe méthylénoxyméthylène et
$R^7$ est hydrogène ou un groupe méthyle.

2. Dérivé de cyclohéxénone de formule I selon la revendication 1, caractérisé par le fait que
A représente le reste $NOR^8$
B, oxygène
X, hydrogène
$R^1$, hydrogène
$R^2$, éthyle ou propyle
$R^3$ et $R^4$ simultanément, brome ou ensemble, époxy,
$R^5$ et $R^6$ simultanément, hydrogène ou ensemble, méthylénoxyéthylène,
$R^7$, hydrogène et
$R^8$, éthyle, allyle, chloropropényle ou butényle.

3. Herbicide selon la revendication 7, caractérisé par le fait que
A représente le reste $NOR^8$
B, oxygène
X, hydrogène
$R^1$, hydrogène
$R^2$, éthyle ou propyle
$R^3$ et $R^4$ simultanément, brome ou ensemble, époxy,
$R^5$ et $R^6$ simultanément, hydrogène ou ensemble, méthylénoxyéthylène,
$R^7$, hydrogène et
$R^8$, éthyle, allyle, chloropropényle ou butényle.

4. Procédé de préparation d'un dérivé de cyclohéxénone de formule I selcn la revendication 1 avec A signifiant oxygène et $R^1$ hydrogène

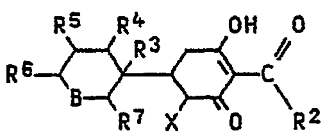

caractérisé par le fait qu'on transforme un composé de formule II, où les restes ont la signification donnée dans la revendication 1

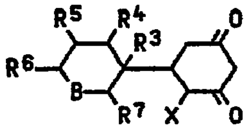

(II),

avec un chlorure d'acide $R^2COCl$ et une base, en l'ester énolique et l'on transpose celui-ci en présence de dérivés d'imidazole ou de pyridine.

5. Procédé de préparation d'un dérivé de cyclohéxénone de formule I selon la revendication 1 avec les significations A = $NOR^8$ et $R^1$ = hydrogène

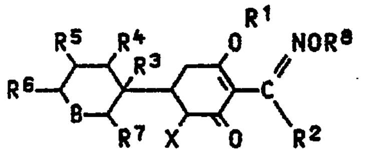

caractérisé par le fait que l'on fait réagir un composé de formule I avec les significations A = oxygène et $R^1$ = hydrogène, avec une O-aliphaténhydroxylamine $R^8ONH_2$, $R^8$ ayant la signification donnée dans la revendication 1.

6. Herbicide contenant un dérivé de cyclohéxénone de formule I selon la revendication 1 avec la signification A = $NOR^8$.

7. Herbicide, contenant un additif inerte et un dérivé de cyclohéxénone de formule I selon la revendication 1 avec A = $NOR^8$.

8. Procédé de lutte contre la croissance des plantes indésirables caractérisé par le fait que l'on traite les plantes indésirables ou les surfaces à maintenir exemptes de croissance de plantes indésirables, avec une quantité efficace du point de vue herbicide d'un dérivé de cyclohéxénone de formule I selon la revendication 1 où A représente un groupe $NOR^8$.